# EUROPEAN PATENT APPLICATION

(11) **EP 2 347 662 A2**
(43) Date of publication of application: **27.07.2011**
(21) Application number: 09826327.0
(22) Date of filing: 13.11.2009
(51) Int. Cl.: A23L 1/302, A23L 1/304, A61P 31/18, A61K 36/06

(54) **KIT OF PHARMACEUTICAL FORMULATIONS CHARACTERISED BY THE PRESENCE OF MOLECULAR OXYGEN**

(30) Priority: 13.11.2008 MX 2008014493
(71) Applicant: Comercializadora S. Car Borr, S.A. de C.V., C.P. 03200 México, D.F (MX)
(72) Inventor: IZQUIERDO ALCALDE, David, C.P. 11595 México, Distrito Federal (MX)
(74) Representative: Saura Cuadrillero, Salvador
(86) International application number: PCT/MX2009/000122
(87) International publication number: WO 2010/056100

(57) **Abstract**

Kit of pharmaceutical formulations **characterised by** the presence of molecular oxygen comprising four elements which in combination furnish benefits for the health of patients with HIV AIDS: i. weight increase of the patient; ii, increase of appetite; iii. reduction of diarrhoea; iv. reduction of the wasting syndrome; v. increase of stability of mood; vi. increase of muscular mass. vii. decrease of opportunistic diseases, in particular infections caused by Candida, herpes virus, cytomegalovirus, Kaposi's sarcoma; viii. prolongation of the period wherein the patient does not require to take antiretrovirals; ix. increase of acceptance of pharmacological treatment; x. reduction of gastritis, nausea or vomiting, headache; xi. reduction of cases with anaemia or thrombocytopenia; xii. a change in the phase of the disease in the patient.

## Description

### TECHNICAL FIELD OF THE INVENTION

This invention relates to a Kit of Pharmaceutical Formulations consisting of molecular oxygen, a vitamin complex, a mineral complex and yeast.

### BACKGROUND

In people infected with HIV AIDS who take multivitamins every day experience a slower progression of their disease, according to a study published in the New England Journal of Medicine.

Dietary supplements "may save time and allow people with HIV to take longer to develop the symptoms requiring antiretroviral therapy", said Lynne Mofensoon, from the National Institute Of Child Health And Human Development which sponsoring the study.

The information was taken from a study started in Tanzania in 1995 with 1000 pregnant women infected with HIV AIDS who participated in an experiment to determine whether vitamin supplements might reduce transmission of the virus to the foetus.

Therefore, a dietary supplement suited to the needs of each patient would lead to weight gain, the correction of nutritional laboratory variables and the resulting improvement in quality of life.

The patient's state of malnutrition due to HIV AIDS is the main cause favouring complications and increasing the patient's morbidity. Therefore, the invention intended for patenting has achieved nutritional support to help improve the patient's condition by improving their BMI and thus also reduce opportunistic infections.

There are no specialised products on the market to help HIV AIDS patients to prevent malnutrition and reduce opportunistic diseases. The closest product, although not comparable, is the product called "Prosure" by Abbot Laboratories, which is indicated for involuntary weight loss, loss of muscle mass, to improve fatigue and quality of life and which is aimed at patients with cancer.

### DESCRIPTION OF THE INVENTION

### GLOSSARY

For a better understanding of this description, we provide a glossary with terms used frequently in this patent application:
- **Molecular Oxygen:** Oxygen that includes water as a diluent medium, such that the oxygen and hydrogen molecules are bound together; One of the essential units in a chemical compound is the molecule, which is the smallest part of a chemical compound acting in a chemical reaction. In most covalent compounds, molecules consist in groups of atoms bound together by coordinate or covalent bonds. Similarly, ionic compounds do not have individual molecules, but are rather formed by a large number of ions of opposite charge.
- **Concentrated Oxygen:** This is an original formulation, which is diluted with an aqueous solution to make it suitable for oral intake.
- **Stabilised Oxygen:** The state of an oxygen formulation in which it maintains its energy distributed in a statistical manner. A state in which the forces, influences, reactions etc., are balanced with one another and in which there are no changes, in other words, the stoichiometry of the formula remains in equilibrium, as long as there are no sudden temperature changes that dissipate and break the equilibrium, producing the loss of oxygen molecules, in this case causing irreversible activity.

### - Health Benefits:

A)Increase in patient weight.
B)Increased appetite.
C) Reduction of diarrhoeas.
D)Reduction of Wasting Syndrome.
E)Increased mood stability.
F)Increasing muscle mass.
G)Decreasing opportunistic diseases, especially infections by Candida, herpes virus, cytomegalovirus, Kaposi's sarcoma.
H)Extending the time during which the patient does not need to take antiretroviral drugs.
I)increased compliance with pharmacological treatments.
J) Decreasing gastritis, nausea or vomiting, headaches.
K)Reduction of cases with anaemia or thrombocytopenia.
L) Changes in the stage of the patient's disease.

### DETAILED DESCRIPTION OF THE INVENTION

A Kit of Pharmaceutical Formulations comprising four components, molecular oxygen, a vitamin complex, a mineral complex and yeast, which together provide health benefits to HIV AIDS patients, such as:
a. Increase in patient weight.
b. Increased appetite.
c. Reduction of diarrhoeas.
d. Reduction of Wasting Syndrome.
e. Increased mood stability.
f. Increased muscle mass.
g. Decreasing opportunistic diseases, especially infections by Candida, herpes virus, cytomegalovirus, Kaposi's sarcoma.
h. Extending the time during which the patient does not need to take antiretroviral drugs.
i. Increased compliance with pharmacological treatments.
j. Decreasing gastritis, nausea or vomiting, headaches.
k. Reduction of cases with anaemia or thrombocytopenia.
l. Changes in the stage of the patient's disease.

When the elements forming the Kit act together, each operates on the human body in the most efficient manner for the parameter they are formulated for. The effectiveness of all four components together is much greater than if dosed separately.

The first of the components in the Kit is characterised by the Molecular Oxygen that can be assimilated orally by the body. HIV AIDS patients require more oxygen as a nutrient and it has been proven that this element is a determinant factor for health.

The stabilised molecular oxygen, supplied as the first nutritional supplement, acts rapidly in the body, since it passes into the bloodstream in its molecular form and is thus distributed to all the cells performing the primary biochemical functions for its use, therefore strengthening the immune system by providing a greater amount of this vital element for subsequent reactions.

This first element is in a liquid state, consisting mainly of molecular oxygen, and consists in:
54% Concentrated Oxygen
(NaCl, B203, NaClO, Na2SO4, NaClO3)
46 % Twice-distilled water

The second element consists in a vitamin complex including: Vitamins C, E, B1 B2, B-Complex, folic acid, Beta Carotene and other compounds that are necessary and essential to the human body, with particular importance given to those lacking in HIV AIDS patients.

The second granulate element consists in a Vitamin Complex, consisting of:
67 % Muscovado Sugar
0.01% Potassium K Sorbate
0.01% Ascorbic acid
0.01% Citric acid
0.03% Acetylsalicylic acid
0^{.}01% Folic acid
0.01% Vitamin 11 Vitamin A Palmitate
0.01% Vitamin E Alpha tocopherol succinate
0.01% Vitamin B1 Thiamin
0.01% Vitamin B2
0.01% Vitamin B3
0.02% Vitamin B6 Pyridoxine
0.01 % Vitamin B12 Cyanocobalamin
0.01% Beta Carotene
13.4% Nutrifort, of which:
   0.001% Phenylalanine
   0.001% Isoleucine
   0.001% Leucine
   0.001% Lysine
   0.001% Methionine
   0.001% Threonine
   0.001% Tryptophan
   0.001% Valine
   0.001% Glycine
   0.001% Glutamic acid
   0.001% Alanin
   0.0001% Aspartic acid
   0.0001% Histidine
   0.0001% Proline
   0.0001% Tyrosine
   0.0001% Arginine
   0.0001% Cysteine
   0.0001% Serine
0.04% Sodium Chloride Salt
0.01% Zinc Sulphate
0.03% L-lysine

The third element consists in a mineral complex containing: Calcium, Iron, Cobalt, Sulphur, Molybdenum, Magnesium, Potassium, Iodine, amongst others, all these supplied on a solid base that is easily absorbed by the body.

Calcium's role in the blood, for example, provides neuromuscular excitability and blood coagulation. The ions act on a certain group of enzymes and the role of electrolytes is vital in the regulation of the acid-base balance of cells. Normal ossification requires a normal ratio between the potassium and calcium in the extracellular fluid, which must be maintained in order to ensure the normal activity of the muscles.

The third liquid element in the mineral complex consists of:
99.90% Twice-distilled water
0.006% Prepared Oxygen
0.002% Citric acid
0.003% Concentrated flavour enhancer

The fourth element consists in a hydroalcoholic extract of unfermented yeast cells, separated by barium sulphate precipitation. This supplement leads to pantothenic acid, biotin and aneurin, and, combined with a complete polysaccharide that provides support to tissues, cartilage, corneas, mucosa, amongst others.

The fourth liquid element is the yeast, consisting of:
98.60% Water
0.008% Acetic acid diluted to 5% acidity
0.003% 85% USP Phosphoric acid
0.0001% Sodium benzoate
0.013% Saccharomyces cerevisiae yeast
0.003% Prepared Oxygen

What is innovative in this pharmaceutical composition is the Oxygen, since there is no dietary supplement with oxygen that allows the human body to obtain more energy by the cells performing the combustion process in the presence of Oxygen and Carbon dioxide, thus favouring the biochemical conditions for obtaining more energy.

We also know that HIV-AIDS patients have greater requirements of oxygen as a nutrient. Since there is a deficiency thereof this can cause almost all degenerative diseases in the body.

Once administered, the body acts rapidly, since it is carried into the bloodstream in its molecular form to be distributed to all the cells that perform the primary biochemical functions requiring it, which therefore strengthens the immune system by providing more of this vital element.

The body's capacity to combat the HIV virus depends absolutely on an effective immune system as well as depending largely on a high, continuous and pure supply of oxygen. It has been proven that stabilised oxygen supplied to the body strengthens the immune system, especially against neoplasias.

The main difference with other products is the presence of oxygen that can be assimilated orally by the body, which allows the body to change the oxidation-reduction reactions, improving its concentration in the tissues and increasing the anaerobic concentrations that prevent the progression of viral infections. It decreases the production of free radicals that cause tissue damage.

### EXAMPLES

The following examples are provided to illustrate the invention, but in no case to limit it.

The use of adequate dietary support together with antiretroviral therapy tend to produce an earlier stabilisation response of the disease, and therefore to improve the patient's quality of life.

If the pharmaceutical composition is taken as a dietary supplement, it provides great benefits to patients and can be administered as follows:
a. An amount of 1 ml in water, three times a day, of the first liquid element consisting of Molecular Oxygen.
b. An amount of 8 g, three times a day, of the second granulate element consisting of the Vitamin Complex.
c. An amount of ml, three times a day, of the third element consisting of the Mineral Complex.
d. An amount of 5 ml, three times a day, of the fourth element consisting in the Yeast.

## Claims

1. A kit of pharmaceutical formulation **characterised in that** it consists in the presence of molecular oxygen, consisting of four components, which when joined provide benefits to patients with HIV AIDS.

2. According to claim 1, the first element in the Kit **characterised in that** it consists in the molecular oxygen, said element consisting of:
54% Concentrated Oxygen
(NaCl, B203, NaClO, Na2SO4, NaClO3)
46% Twice-distilled water

3. According to claim 1, the second element in the Kit is **characterised in that** it comprises the Vitamin Complex, containing:
67 % Muscovado Sugar
0.01% Potassium K Sorbate
0.01% Ascorbic acid
0.01% Citric acid
0.03% Acetylsalicylic acid
0.01% Folic acid
0.01% Vitamin A Vitamin A Palmitate
0.01% Vitamin E Alpha tocopherol succinate
0.01% Vitamin B1 Thiamin
0.01% Vitamin B2
0.01% Vitamin B3
0.02% Vitamin B6 Pyridoxine
-0.01 % Vitamin B12 Cyanocobalamin
0.01% Beta Carotene
13.4% Nutrifort, of which:
0.001% Phenylalanine
0.001% Isoleucine
0.001% Leucine
0.001% Lysine
0.001% Methionine
0.001% Threonine
0.001% Tryptophan
0.001% Valine
0.001% Glycine
0.001% Glutamic acid
0.001% Alanin
0.0001% Aspartic acid
0.0001% Histidine
0.0001% Proline
0.0001% Tyrosine
0.0001% Arginine
0.0001% Cysteine
0.0001% Serine
0.04% Sodium Chloride Salt
0.01% Zinc Sulphate
0.03% L-lysine

4. According to claim 1, the third element in the Kit is **characterised in that** it comprises the Mineral Complex, containing:
99.9% Twice-distilled water
0,006% Prepared Oxygen
0.002% citric acid
0.003% Flavour enhancer

5. According to claim 1, the fourth element in the Kit comprises the Yeast, and contains:
98.6% Waster
0.008% Acetic acid diluted to 5% acidity
0.003% 85% USP Phosphoric acid
0.0001% Sodium benzoate
0.013% Yeast (Saccharomyces cerevisiae)
0.003% Prepared Oxygen

6. The mode of use of the kit according to claim 1 in order to obtain health benefits for patients with HIV AIDS is **characterised by** its form of administration, which is described below:
A. An amount of 1 ml in water, three times a day, of the first liquid element consisting of Molecular Oxygen.
B. An amount of 8 g, three times a day, of the second granulate element consisting of the Vitamin Complex.
C. An amount of 5 ml, three times a day, of the third liquid element consisting of the Mineral Complex.
D. An amount of 5 ml, three times a day, of the fourth liquid element consisting in the Yeast.
